# EUROPEAN PATENT SPECIFICATION

(11) **EP 3 152 576 B1**
(45) Date of publication and mention of the grant of the patent: **12.09.2018**
(21) Application number: 15726199.1
(22) Date of filing: 05.06.2015
(51) Int. Cl.: G01N 33/574

(54) **USE OF TM9SF4 AS A BIOMARKER FOR TUMOR ASSOCIATED EXOSOMES**
VERWENDUNG VON TM9SF4 ALS BIOMARKER FÜR TUMORASSOZIIERTE EXOSOMEN
UTILISATION DE TM9SF4 COMME BIOMARQUEUR POUR LES EXOSOMES ASSOCIEÉS AUX TUMEURS

(30) Priority: 06.06.2014 EP 14171464
(43) Date of publication of application: 12.04.2017
(73) Proprietor: Exosomics Siena S.p.A., 53100 Siena (IT)
(72) Inventor: LOZUPONE, Francesco, I-00141 Roma (IT); CHIESI, Antonio, I-37068 Vigasio (Verona) (IT); GUAZZI, Paolo, EE-10116 Tallin (EE); ZAROVNI, Natasa, I-20131 Milano (IT); FERRUZZI, Pietro, I-50125 Firenze (IT); ZOCCO, Davide, I-53100 Siena (IT)
(74) Representative: Comoglio, Elena
(86) International application number: PCT/EP2015/062594
(87) International publication number: WO 2015/185730

(56) References cited:
- US-A1- 2012 058 492
- US-A1- 2012 122 118
- EVA OGOREVC ET AL: "The role of extracellular vesicles in phenotypic cancer transformation", RADIOLOGY AND ONCOLOGY, vol. 47, no. 3, 1 January 2013 (2013-01-01), pages 197-205, XP55234317, SI ISSN: 1318-2099, DOI: 10.2478/raon-2013-0037
- O. GALAMB ET AL: "Diagnostic mRNA Expression Patterns of Inflamed, Benign, and Malignant Colorectal Biopsy Specimen and their Correlation with Peripheral Blood Results", CANCER EPIDEMIOLOGY, BIOMARKERS & PREVENTION, vol. 17, no. 10, 1 October 2008 (2008-10-01), pages 2835-2845, XP55008197, ISSN: 1055-9965, DOI: 10.1158/1055-9965.EPI-08-0231

## Description

The present invention relates to extracellular microvesicles biomarkers for determining the tumour transformation status or presence of a tumour in a subject, and to the uses of such biomarkers and to diagnostics methods using such biomarkers.

### BACKGROUND TO THE INVENTION

Contrary to malignant (or cancerous) tumours, benign tumours typically are mass of cells that lack the ability to invade neighbouring tissue or metastasise. Also, benign tumours generally have a slower growth rate than malignant tumours and the tumour cells are usually more differentiated.

Although most benign tumours are not life-threatening, many types of benign tumours have the potential to become cancerous (malignant) through a process known as tumour transformation.

Non Metastatic Cancer (primary or recurrent) is a cancer that has not spread from the primary site (place where it started) to other places in the body.

Metastatic cancer is a cancer that has spread from the part of the body where it started (the primary site) to other parts of the body.

The development of benign neurofibromas can often be linked to a mutation of the NF1 tumor suppressor gene in cells of the Schwann cell lineage¹⁻³. These neoplasms can frequently undergo a further transformation to malignant peripheral nerve sheet tumors (MPNSTs)¹⁻³. It is currently unclear which cell types are particularly susceptible to MPNST formation, which are the molecular changes causing the development of MPNSTs from neurofibromas, or which other factors in the tumor environment might contribute to neoplasia. In addition, gliomas, particularly pilocytic astrocytomas of the optic nerve, and leukemias, are seen with increased frequency in the NF1 population³.

MPNSTs have very poor prognosis as they do not respond to standard chemo- or radiation therapy and have a high propensity to metastasize⁴⁻⁷. NF1 patients and their families are well aware of these facts, which is why the development of an MPNST is the complication that is most dreaded by patients suffering from this disease⁸. However, early detection is often hampered by the fact that MPNSTs frequently develop within preexisting large neurofibromas, making new growth or progression difficult to detect and distinguish even with MRI. This diagnostic delay is likely the cause of poor outcome of MPNST in NF1 with respect to their sporadic counterparts. This constitutes the major impetus for identification of molecular alterations that can be detected in a noninvasive manner and are indicative of MPNST initiation and progression in NF1 patients that would be useful in screening and early diagnosis as well as monitoring of disease or therapeutic outcome in preclinical and clinical settings.

It is generally agreed that multiple neurofibroma subtypes exist which differ in their location and pattern of growth, their association with NF1 and their potential for malignant transformation. Many clinical and basic science investigators broadly classify neurofibromas as either dermal or plexiform variants¹. Plexiform neurofibromas are neurofibroma variants that occur almost exclusively in NF1 patients and are thought to be congenital; they are distinguished from localized intraneural neurofibromas by their characteristic plexiform growth pattern. Plexiform neurofibromas have the highest risk for malignant transformation into MPNST¹.

Similarly to neurofibromas transformation into MPNST, other benign tumors have a risk to transform into their malignant counterpart. This is for example the case of Benign Prostatic Hyperplasia (BPH) to prostate cancer, colon polyps to colorectal cancer, benign nevi to melanoma, non cancerous breast conditions to breast cancer, lung nodules to lung cancer, early stage astrocytoma to glioblastoma, and benign ovarian tumors to ovarian cancer. Most of these cancers are also able to metastatise

Extracellular vesicles (EVs) are a class of membrane bound organelles secreted by various cell types⁹. EVs not limitedly include (i) exosomes: 30-100 nm diameter membraneous vesicles of endocytic origin (ii) ectosomes (also referred to as shedding microvesicles, SMVs): large membranous vesicles (50-1000 nm diameter) that are shed directly from the plasma membrane (PM) and (iii) apoptotic bodies (50-5000 nm diameter): released by dying cells.

Exosomes are natural lipidic extra cellular nanovescicles produced and released by virtually all cell types in a finely regulated and functionally relevant manner so that the protein and mRNA composition reflects the type and condition of a parent cell¹⁰⁻¹⁴. These vesicles have intrinsic stability and ability to cross biological barriers, so that exosomes originated from different tissues can be found in easily accessible biological fluids such as blood¹⁵⁻¹⁷. Given their biological roles and features, exosomes are considered early sentinels of alterations in cell and tissue homeostasis and metabolism and are an appealing source for identification of novel disease-relevant biomarkers as well as display of known tissue markers in a liquid biopsy paradigm. This is a major premise and promise of using exosome targeted assays in diagnostics of complex diseases such as cancer. The major challenge lies in association of exosome associated markers, both proteins and RNAs, to a particular tissue, in a particular condition and optimization of reliable, affordable, noninvasive exosome targeted solutions and assays that can be realistically implemented in clinical research and practice¹⁸⁻²¹.

There currently is a need for extracellular vesicle biomarkers that are able to determine the presence of a tumour (be it benign, malignant and metastatic) or the transformation status of a tumour (benign to malignant and non-metastatic to metastatic)

**TM9SF4 protein (SEQ ID NO: 1)** is a recently described transmembrane protein that belongs to Transmembrane-9 Superfamily (TM9SF), a well-defined family of proteins characterized by a large hydrophylic N-terminal domain followed by nine transmembrane domains²². This protein is known to be overexpressed in melanoma and in acute myeloid leukemia and myelodysplastic syndromes, latter due to a three to tenfold amplification of a chromosome 20 fragment (20q11.21) bearing the entire *TM9SF4* gene^{23,24}. TM9SF4 is involved in phagocytosis of bacteria and in the cannibal phenotype of metastatic melanoma cells, a phenomenon often related with poor prognosis^{25,26}. Cannibal cancer cells have been frequently detected in gastric and colon cancers²⁷⁻³⁰

It has been recently shown that TM9SF4 binds to V-ATPase, a pH regulating proton pump overexpressed in several tumors. This interaction aberrantly stabilizes the proton pump in its active state with the consequent pH gradient alterations that in turn is associated with drug resistance and invasiveness of colon cancer cells³¹.

**CD9 protein (SEQ ID NO: 2)** is a member of the transmembrane 4 superfamily, also known as the tetraspanin family. Tetraspanins are cell surface glycoproteins with four transmembrane domains that form multimeric complexes with other cell surface proteins. The encoded protein functions in many cellular processes including differentiation, adhesion, and signal transduction, and expression of this gene plays a critical role in the suppression of cancer cell motility and metastasis. It is found on the surface of exosomes and is considered exosome housekeeping protein for the quantitative analysis of plasma derived nanovesicles.

**miRNA21 (SEQ ID NO: 3)** miRNAs are a class of small non-coding RNAs whose mature products are ∼22 nucleotides long. They negatively regulate gene expression by inducing translational inhibition or transcript degradation³². miR-21 has been found to be upregulated in many pathological conditions including cancer and cardiovascular diseases³³. The identification of several targets of miRNAs which are actually classical oncogenes or tumor suppressors has led to the widely accepted idea that miRNAs play pivotal roles in cancer initiation, progression and metastasization^{34,35} miR-21 was first noted as an apoptotic suppressor in various cell lines³⁶.

**RNU6 (SEQ ID NO: 4)** is a non-coding RNA (ncRNA) molecule which functions in the modification of other small nuclear RNAs (snRNAs). Accurate profiling of microRNAs (miRNAs) is an essential step for understanding the functional significance of these small RNAs in both physiological and pathological processes. It is well-known that normalization is one of the most critical steps in qRT-PCR and commonly used genes for this purpose, such as U6 and 5S³⁷, have already been described as being differentially expressed in cancer, which makes these genes not suitable as internal controls.

### DESCRIPTION OF THE INVENTION

In a first aspect of this invention, there is provided a method for determining in vitro the presence of a tumour in a subject, such method comprising:
a) providing a biological sample obtained from that subject,
b) isolating extracellular vesicles from said sample, wherein this step of isolating extracellular vesicles comprises isolating TM9SF4-positive extracellular vesicles,
c) determining, from the extracellular vesicles isolated in step b), the level or presence of a suitable biomarker, and
d) comparing the level or presence of the biomarker determined in step c) with one or more reference values,
characterized in that the TM9SF4-positive extracellular vesicles are isolated through binding to an anti-TM9SF4 antibody.

In another embodiment, at least a portion of the extracellular vesicles are exosomes.

In one embodiment, the tumour is colon cancer.

In another embodiment, the tumour is gastric cancer.

In another embodiment, the tumour is breast cancer.

In another embodiment, the tumour is lung cancer.

In another embodiment, the tumour is melanoma.

In another embodiment, the tumour is pancreatic cancer.

In another embodiment, the tumour is ovary cancer.

In another embodiment, the tumour is prostate cancer.

In another embodiment the tumour is a central nervous system tumour.

In a particular embodiment, the central nervous system tumour is glioblastoma.

In another embodiment, the tumour is MPNST.

In one embodiment, the biomarker of step c) is CD9 protein.
In another embodiment, the biomarker of step c) is miR-21.

In another embodiment, the biomarker of step c) is RNU6.

Any combination of the above embodiments of this first aspect of the invention represent further embodiments of the invention.

In a second aspect to this invention, there is provided a method for determining in vitro the tumour transformation status in a subject, such method comprising:
a) providing a biological sample obtained from that subject,
b) isolating extracellular vesicles from said sample, wherein this step of isolating extracellular vesicles comprises isolating TM9SF4-positive extracellular vesicles,
c) determining, from the extracellular vesicles isolated in step b), the level or presence of a suitable biomarker, and
d) comparing the level or presence of the biomarker determined in step c) with one or more reference values,
characterized in that the TM9SF4-positive extracellular vesicles are isolated through binding to an anti-TM9SF4 antibody.

In one embodiment, the biological sample of step a) is obtained from a patient affected by a benign tumour.

In another embodiment, at least a portion of the extracellular vesicles are exosomes.

In one embodiment the tumour transformation status is the transformation to an MPNST.

In another embodiment, the tumour transformation status is the transformation to a colorectal cancer.

In one embodiment, the biomarker of step c) is CD9 protein.

In another embodiment, the biomarker of step c) is miR-21.

In another embodiment, the biomarker of step c) is RNU6.

Any combination of the above embodiments of this second aspect of the invention represent further embodiments of the invention.

A third aspect of this invention concerns the use of TM9SF4-positive extracellular vesicles in a test to determine the presence of a tumour or the tumour transformation status in a subject characterized in that the presence of the tumour or the tumour transformation status is determined by a method under the first or second aspect this invention

In one embodiment, at least a portion of the extracellular vesicles are exosomes.

In one embodiment, the tumour is colon cancer.

In another embodiment, the tumour is gastric cancer.

In another embodiment, the tumour is breast cancer.

In another embodiment, the tumour is lung cancer.

In another embodiment, the tumour is melanoma.

In another embodiment, the tumour is pancreatic cancer.

In another embodiment, the tumour is ovary cancer.
In another embodiment, the tumour is prostate cancer.

In another embodiment the tumour is a central nervous system tumour.

In a particular embodiment, the central nervous system tumour is glioblastoma.

In another embodiment, the tumour is MPNST.

In one embodiment the tumour transformation status is the transformation to an MPNST.

In another embodiment, the tumour transformation status is the transformation to a colorectal cancer.

Any combination of the above embodiments of this third aspect of the invention represent further embodiments of the invention.

In a fourth aspect of this invention, there is provided the use of a kit to determine the presence of a tumour or a tumour transformation status in a subject, such kit comprising an anti-TM9SF4 antibody and a reagent selected from the list consisting of an anti CD9-antibody, a miR-21 primer or a RNU6 primer

In one embodiment, the tumour is colon cancer.

In another embodiment, the tumour is gastric cancer.

In another embodiment, the tumour is breast cancer.

In another embodiment, the tumour is lung cancer.

In another embodiment, the tumour is melanoma.

In another embodiment, the tumour is pancreatic cancer.

In another embodiment, the tumour is ovary cancer.

In another embodiment, the tumour is prostate cancer.

In another embodiment the tumour is a central nervous system tumour.

In a particular embodiment, the central nervous system tumour is glioblastoma.

In another embodiment, the tumour is MPNST.

In one embodiment the tumour transformation status is the transformation to an MPNST.

In another embodiment, the tumour transformation status is the transformation to a colorectal cancer.

In another embodiment, the kit further comprises instructions for suitable operational parameters in the form of a label or separate insert.

Any combination of the above embodiments of this fourth aspect of the invention represent further embodiments of the invention.

In all aspects and embodiments of the present invention, a "biological sample " is for example a tumor sample, a bodily fluid, a plasma sample ,a urine sample or a saliva sample

In all aspects and embodiments of the present invention, a " subject" is either a human or a mammal.

Due to the micellar nature of extracellular vesicles such as exosomes, some biomolecules present in these vesicles can be detected without lysing the vesicles because they reside on the membrane, whereas some others may only be detected after lysis of the vesicles because they are located within the vesicle.

| Biomarker | Type | Detected from |
|---|---|---|
| CD9 | Protein | Extracellular vesicle membrane |
| miR-21 | miRNA | Whole Extracellular vesicle |
| RNU6 | snRNA | Whole Extracellular vesicle |

### EXAMPLES

There now follows by way of example only a detailed description of the present invention with reference to the accompanying drawings, in which:

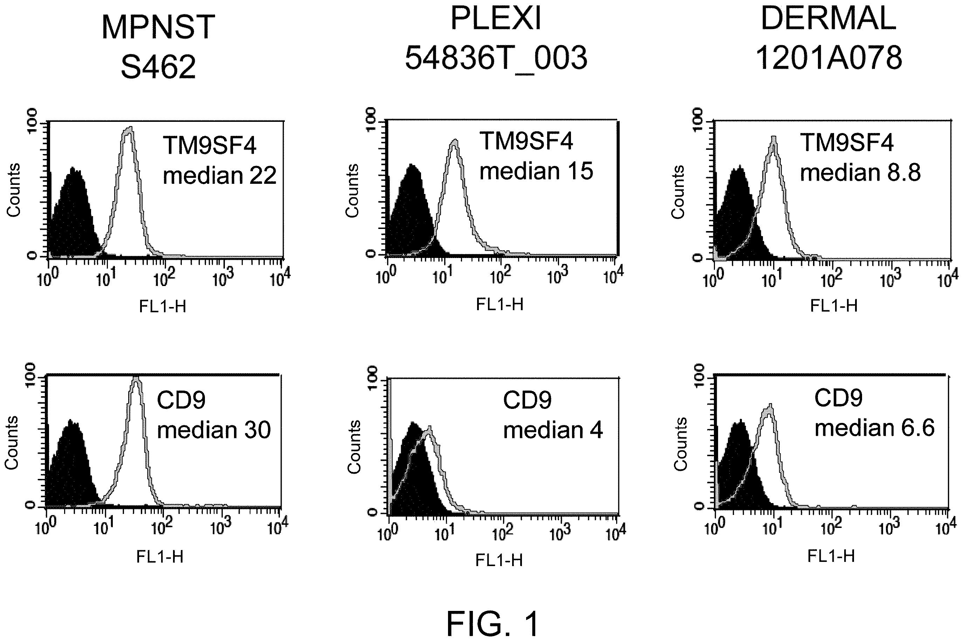

Fig. 1 compares the levels of biomarkers TM9SF4 and CD9 measured by FACS on an MPNST cell line (S462, first column), a Plexiform Neurofibroma line (54836T_003, second column) and a dermal neurofibroma cell line (1201A078, third column). The median values demonstrate that the biomarkers, when detected from the exosome membrane, can differentiate between benign (plexiform neurofibroma, dermal neurofibroma) and malignant (MPNST) conditions.

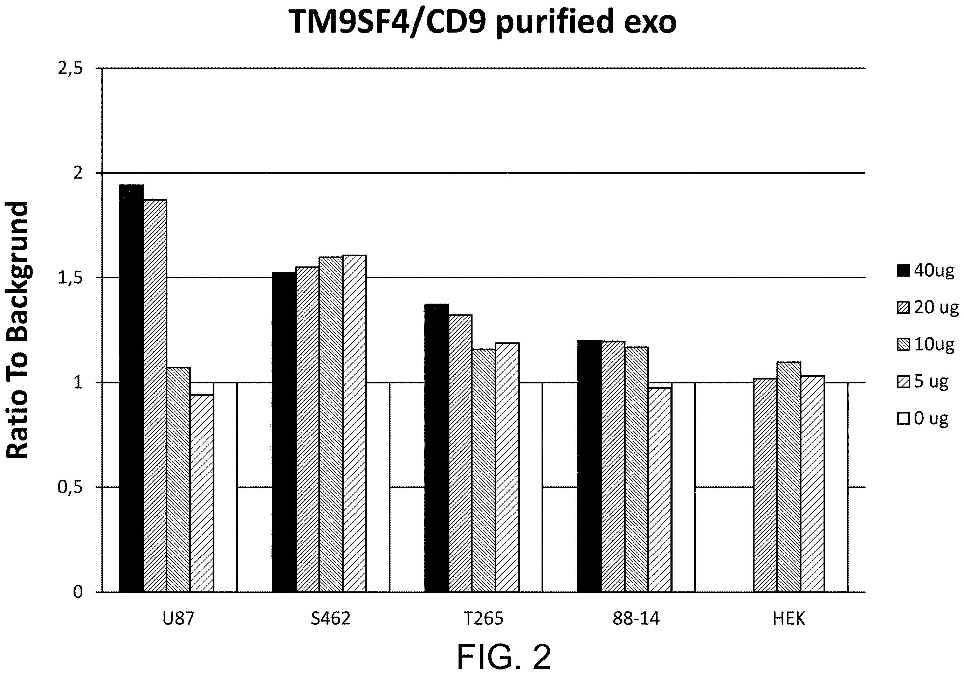

Fig. 2 shows the results of a sandwich Elisa test where 40, 20, 10 and 5 µg of exosomes purified by ultracentrifugation protocol from conditioned media originating from a glioblastoma cell line (U87) or three MPNST cell lines (S462, T265 and 88-14) or from a human embryonic kidney cell line (HEK293) are captured with an anti-TM9SF4 antibody and detected with an anti-CD9 antibody, showing that these biomarkers are expressed on exosomal membrane and that this particular sandwich Elisa assay can be used to detect malignant neurofibroma (MPNST) or other solid tumors (for ex Glioblastoma) derived exosomes and not HEK293 purified exosomes. Ratio to Background reported in the ordinate axis correspond to the absorbance values of each sample divided for the background average absorbance (PBS alone, 0 µg= Ratio to Background 1).

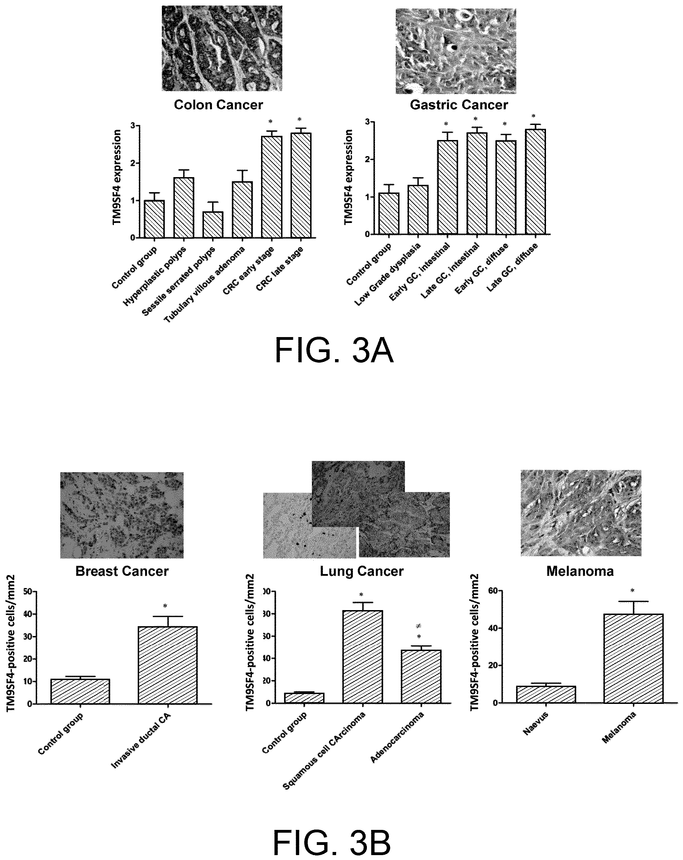

Fig 3A. IHC assessment of TM9SF4 in subjects with Colorectal cancer (CRC) and gatric cancer(GC) compared to healthy surrounding tissue and pre-neoplastic lesions (hyperplastic polyps and tubullovillous adenoma, and gastric dysplasia respectively), revealed highly specific staining of tumor tissue in both early and advanced stages, with no or little expression in healthy or dysplastic tissue. Overall 90% of cancers examined strongly expressed TM9SF4 and the level of expression (IHC score) significantly correlated with disease stage. Fig.3B IHC staining of TM9SF4 positive cells/mm2 in breast, lung and melanoma cancers compared to healthy surrounding tissues. The figure revealed a significant higher number of TM9SF4 positive cells/mm2 in all the cancer tissues analyzed.

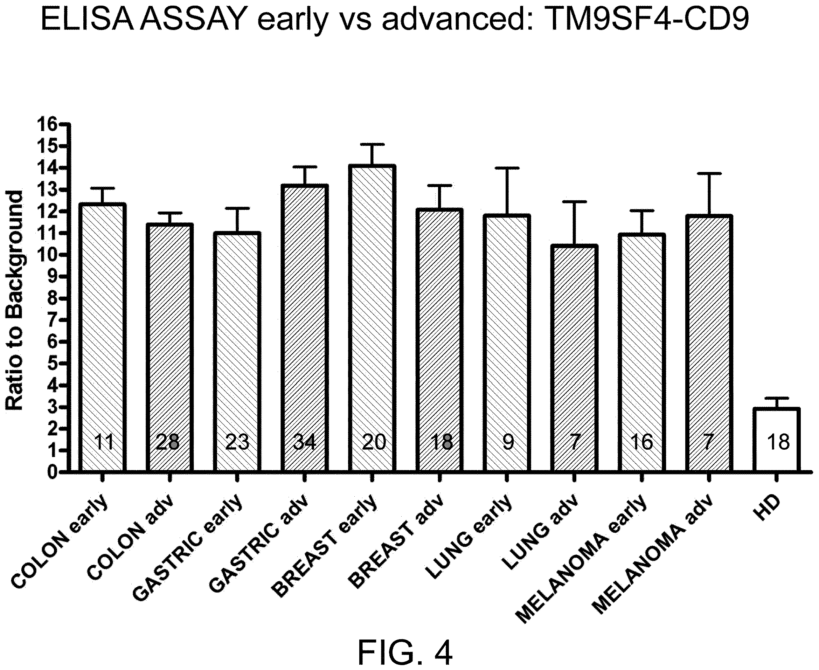

Fig 4 shows the results of a sandwich ELISA test where 100 µl of pre-cleared (see materials and methods) plasma samples obtained from early (TNM classification T1-2N0M0) or advanced (TNM classification T3-4NxMx) tumoral stage patients have been immune-captured through TM9SF4 antibody coated 96 well plates. The detection by CD9 antibody revealed highly specific Ratio to Background values of tumor plasma samples in both early and advanced stages, with very low expression in healthy donors plasma samples. The numbers in the bar-graph corresponded to the number of observations for each study group. Ratio to Background was calculated by dividing samples absorbance values for the background value (only PBS in the well Ratio to Background = 1).

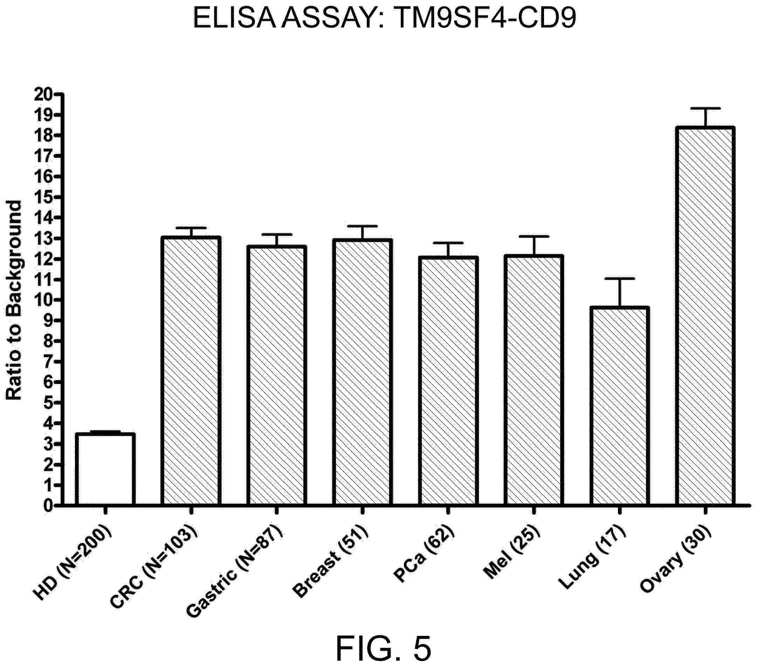

Fig. 5 shows the results of a sandwich ELISA test where 100 µl of pre-cleared (see materials and methods) plasma samples obtained from tumoral patients have been immune-captured through TM9SF4 antibody coated 96 well plates. The detection by CD9 antibody revealed highly specific Ratio to Background values of tumor plasma samples with very low expression in healthy donor plasma samples. In the horizontal axis is reported the tumor group and the number of observations (N). Ratio to Background was calculated by dividing samples adsorbance values for the background value (only PBS in the well Ratio to Background =1).

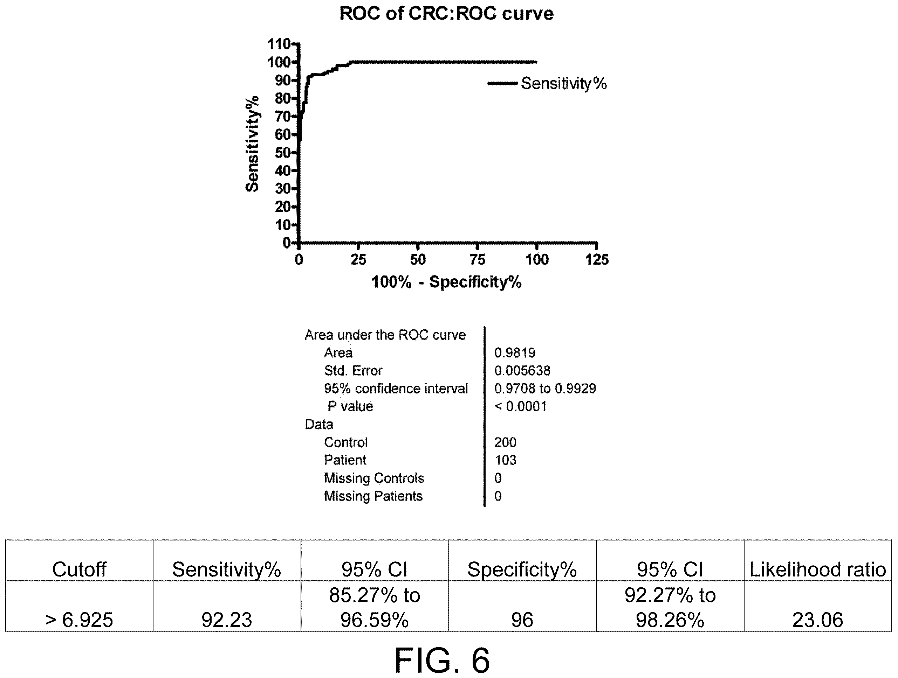

Fig. 6 represents a Receiver Operating Characteristic (ROC) curve calculated by GraphPad Prism program using the Colorectal Cancer (CRC) data reported in Figure 5. Healthy Donor group was used to calculate the specificity and the optimal threshold of TM9SF4/CD9 ELISA sandwich assay on plasma samples. The figure shows how assuming a threshold of >6.925 the test has a sensitivity >92% and a specificity >95%.

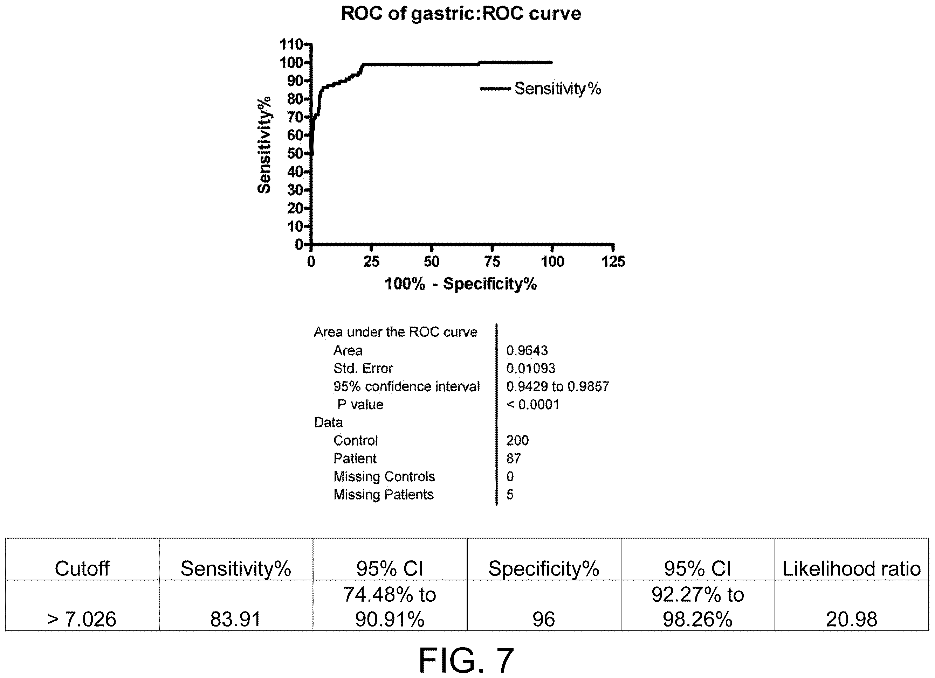

Fig. 7 represents a ROC curve calculated by GraphPad Prism program using the Gastric Cancer data reported in Figure 5. Healthy Donor group was used to calculate the specificity and the optimal threshold of TM9SF4/CD9 ELISA sandwich assay on plasma samples. The figure shows how assuming a threshold >7.025 the test has a sensitivity >83.9% and a specificity >95%.

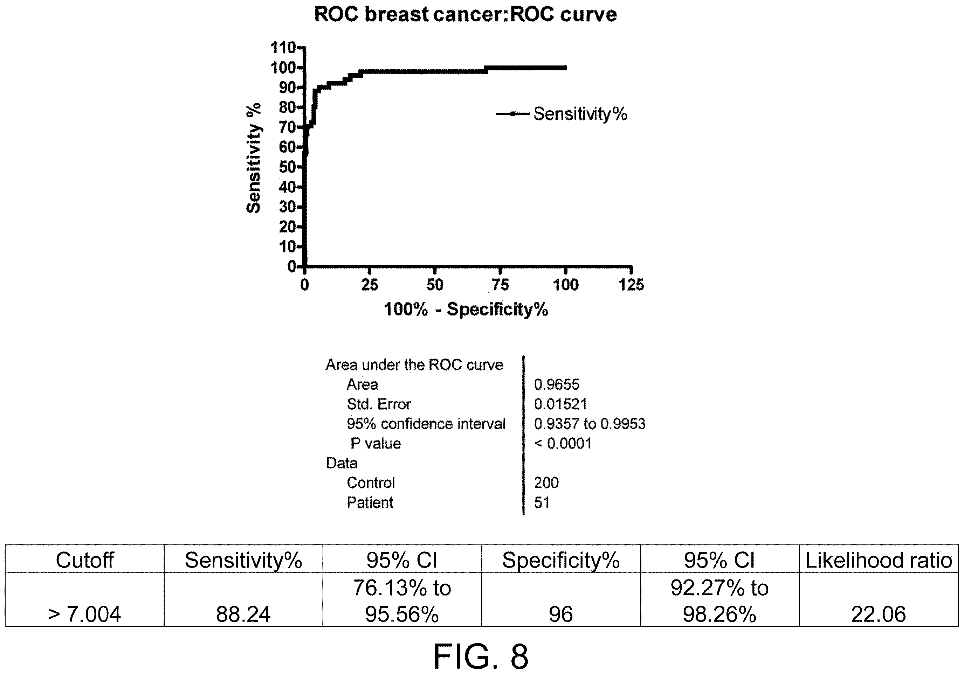

Fig.8 represents a ROC curve calculated by GraphPad Prism program using the Breast Cancer data reported in Figure 5. Healthy Donor group was used to calculate the specificity and the optimal threshold of TM9SF4/CD9 ELISA sandwich assay on plasma samples. The figure shows how assuming a threshold >7.004 the test has a sensitivity >88.2% and a specificity >95%.

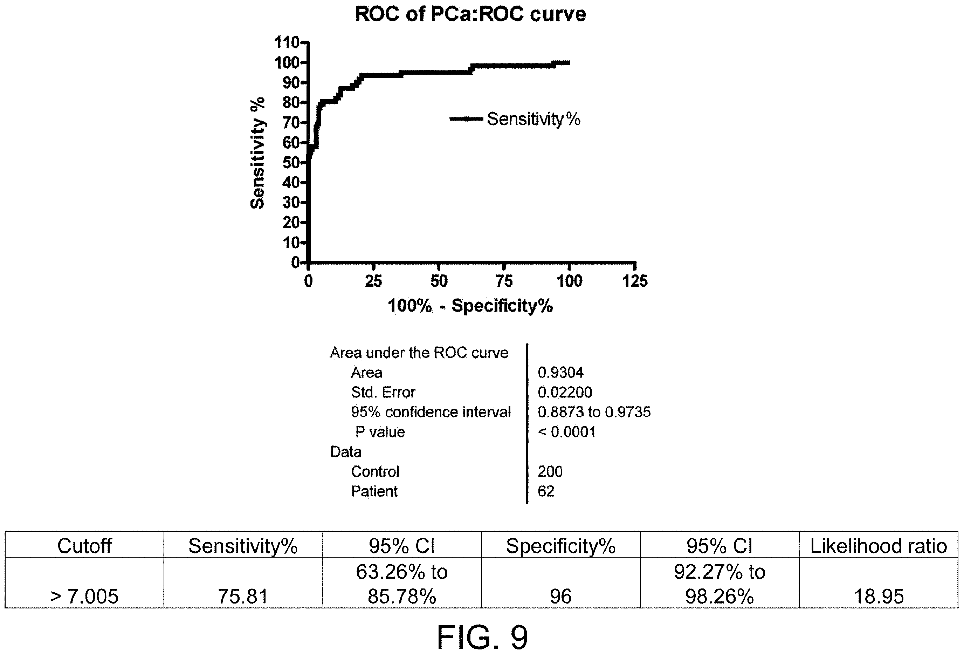

Fig.9 represents a ROC curve calculated by GraphPad Prism program using the Prostate Cancer data reported in Figure 5. Healthy Donor group was used to calculate the specificity and the optimal threshold of TM9SF4/CD9 ELISA sandwich assay on plasma samples. The figure shows how assuming a threshold >7.005 the test has a sensitivity >75.8% and a specificity >95%.

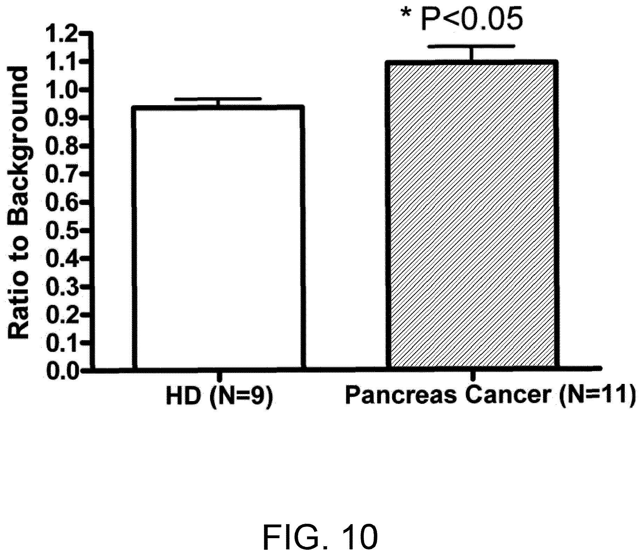

Fig. 10 shows the results of a sandwich ELISA test where 100 µl of pre-cleared (see materials and methods) SERUM samples obtained from tumoral patients have been immune-captured through TM9SF4 antibody coated 96 well plates. The detection by CD9 antibody revealed a significant higher Ratio to Background values of tumor serum samples when compared to healthy donor serum samples. These results suggest that the test ELISA TM9SF4/CD9 is suitable also for Pancreas Cancer plasma samples.

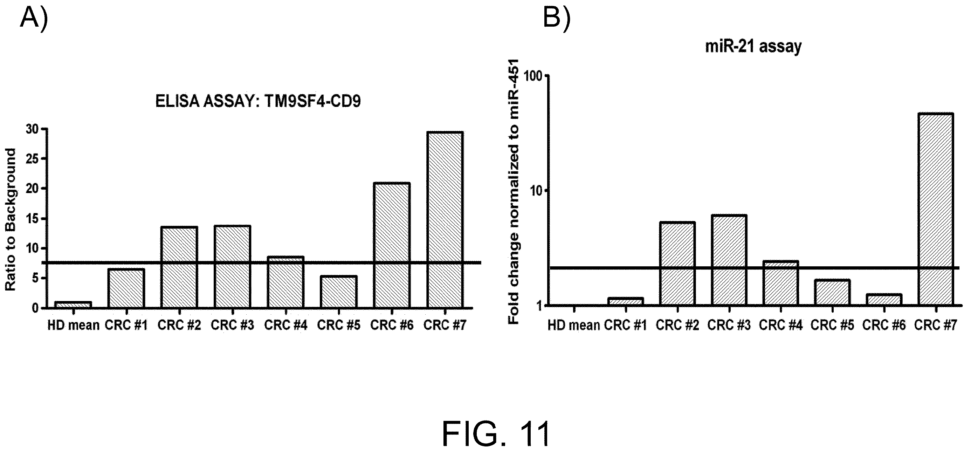

Fig. 11-A shows the results of a sandwich ELISA test where 100 µl of pre-cleared (see materials and methods) plasma samples obtained from seven colorectal cancer (CRC #1-#7) and control group (healthy donors -HD) have been immune-captured through TM9SF4 antibody coated 96 well plates. Figure 11-B shows the relative expression of extracellular vesicle-(EV)-derived miR-21 (normalized to miR-451) from 100 µl of the SAME set of samples. The TM9SF4-positive vesicles were captured using anti-TM9SF4-antibody-coated beads and RNA was extracted and analyzed by RT-qPCR as described in the Material and Methods section. The diagnostic threshold (horizontal line) for the ELISA assay was set as previously described (see materials and methods), and for the miR-21 assay set at a value 2-fold greater than the mean value of the control group. Surprisingly, 6 out of 7 CRC samples showed matched diagnostic results, suggesting a correlation between these two TM9SF4-immunocapture-based assays.

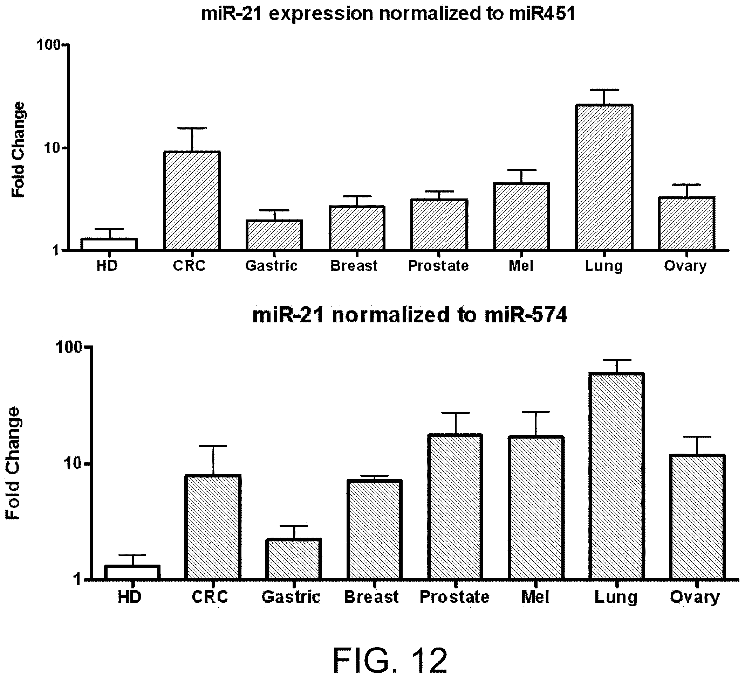

Fig. 12 shows the relative expression of EV-derived miR-21 (normalized to miR-451 or to miR-574) from 100 µl of plasma from cancer patients (Colorectal Cancer (CRC) N = 7; Gastric Cancer N = 6; Breast Cancer N = 6; Prostate Disease N = 5; Melanoma N = 5; Ovary N = 6; Lung Cancer N= 6) and control group (healthy donors N = 11). The TM9SF4-positive EVs were captured using anti-TM9SF4-antibody-coated beads and RNA was extracted and analyzed by RT-qPCR as described in the Material and Methods section. The data suggest that EV-derived miR-21 is over-expressed in the plasma of cancer patients and that both miR-451 and miR-574 are suitable reference miRNAs for determining the relative expression of tumor-derived miRNAs from EVs.

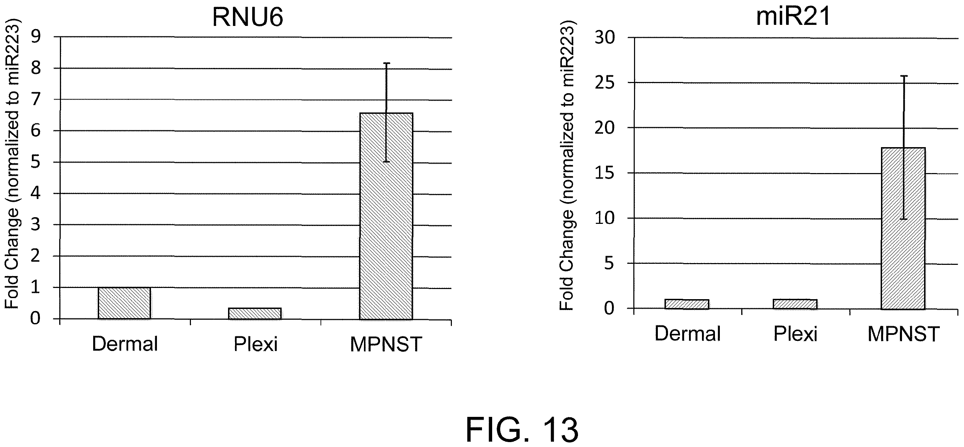

Fig. 13 shows the relative expression of EV-derived RNU6 and EV-derived miR-21 (normalized to miR-223) from 1ml of concentrated (10X) cell supernatant from dermal, plexiform and MPNST cell lines. The TM9SF4-positive EVs were captured using anti-TM9SF4-antibody-coated beads and RNA was extracted and analyzed by RT-qPCR as described in the Material and Methods section. The data suggest that EV-derived RNU6 and miR-21 are over-expressed in the supernatant of human cancer cell lines (MPNST) but not in the supernatant of benign tumor-derived cell lines (plexiform) or normal cell lines (dermal).

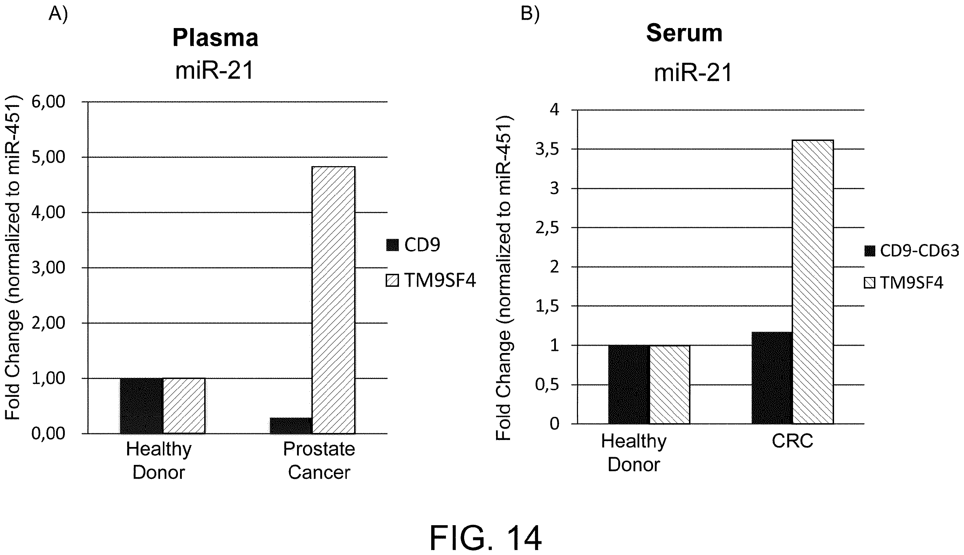

Fig. 14-A shows the relative expression of EV-derived miR-21 (normalized to miR-451) from 100 µl of plasma from a prostate cancer patient and a healthy donor. The EVs were captured using anti-CD9-antibody-coated beads or anti-TM9SF4-antibody-coated beads. RNA was extracted and analyzed by RT-qPCR as described in the Material and Methods section. Figure 14-B shows the relative expression of EV-derived miR-21 (normalized to miR-451) from 100 µl of serum from a colorectal cancer (CRC) patient and a healthy donor. The EVs were captured using beads coated with both anti-CD9 and anti-CD63 antibodies or anti-TM9SF4-antibody-coated beads and RNA was extracted and analyzed by RT-qPCR as described in the Material and Methods section. The data from Fig 14-A and -B suggest that immunocapture of tumor-derived EVs with anti-TM9SF4-antibody-coated beads enriches for miR-21 (a well-known cancer-associate miRNA) in BOTH plasma and serum. Conversely EV capture with antibodies targeting generic EV-markers (CD9 or CD63) does not enrich for miR-21.

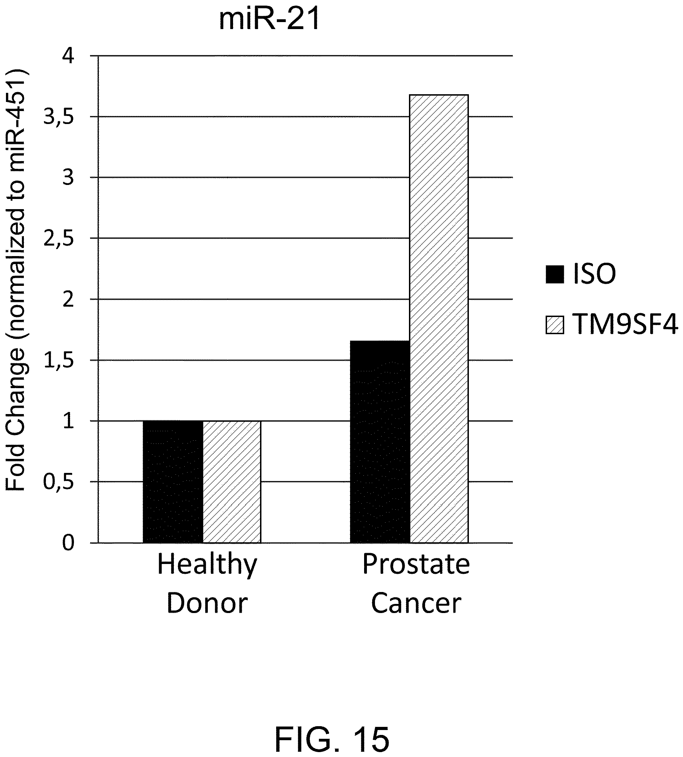

Fig. 15 shows the relative expression of EV-derived miR-21 (normalized to miR-451) from 100 µl of plasma from a prostate cancer patient and a healthy donor. The EVs were captured using anti-TM9SF4-antibody-coated beads or beads coated with isotype-matched-IgG antibodies (ISO) for assessing aspecific binding. RNA was extracted and analyzed by RT-qPCR as described in the Material and Methods section. The data shows the specific enrichment of TM9SF4-positive-EVs using anti-TM9SF4-Ab-coated beads while low aspecific binding was observed in the plasma of the cancer patient.

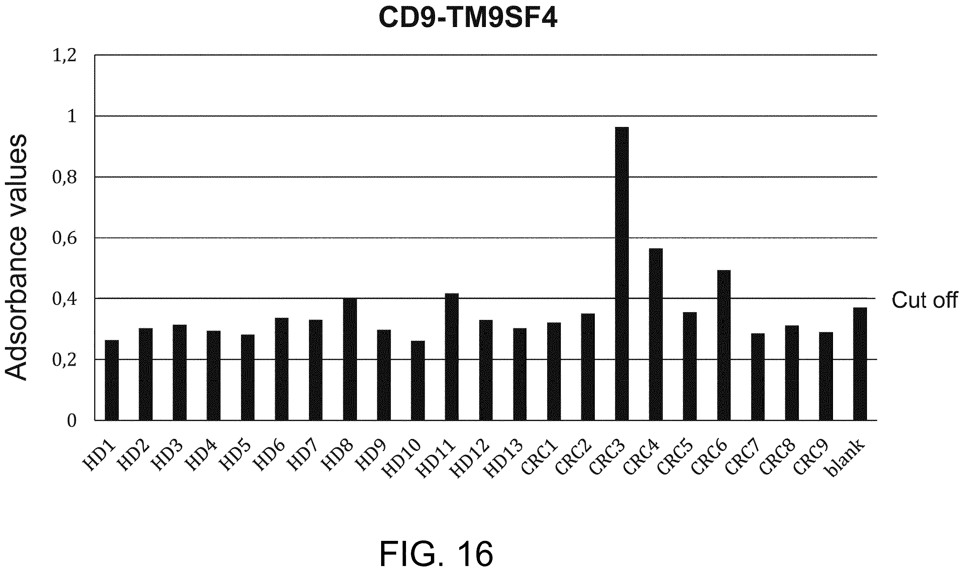

Fig 16 shows the results of a sandwich ELISA test where 100 µl of pre-cleared (see materials and methods) plasma samples obtained from tumoral patients and healthy donors have been immune-captured through CD9 antibody coated 96 well plates. The detection by TM9SF4 antibody revealed that inverting the capture and detection antibody used in Fig. 5 is not useful to distinguish tumoral origin plasma samples from healthy donor plasma samples. Ratio to Background was calculated by dividing samples adsorbance values for the background value (only PBS in the well Ratio to Background =1).

### METHODS

What follows is a description of the methods used in the examples for isolating and analysing the exosomes. The skilled man in the art will recognise that alternative, equivalent, methods exist.

### Exosome isolation by Ultracentrifugation protocol.

Conditioned medium for exosome preparation and analysis should be collected from 80-90% confluent cells of interest.

Supernatant from cell culture are collected in sterile conditions and added with protease inhibitors diluted 1:1.000, pre-cleared by filtration (0.2 µm), and Ultracentrifuged (ca. 50 mL/tube) at 110.000 g for 1.5 hour at +4°C. The supernatant is then removed and discarded. The pellet is re-suspend in 100 µl of ice cold PBS before dilution in 50 mL ice cold 1xPBS and ultracentrifuged at 110.000 g for 1.5 hour at +4°C. The resulting pellet is re-suspended in 100 µl PBS and vortexed for 30 seconds before pipetting for experimentation.

### Standard protocol for protein markers detection by FACS analysis

Exosomal concentration is quantified using Bradford method for protein quantification. Exosomes isolated from cell lines supernatants are incubated at 4°C over night with aldehyde/sulfate latex beads (4% w/v, 4 µm) in 1:20 ratio. After a washing step in PBS, the exosomes adsorbed on beads surface are incubated in PBS+0.5% BSA with relevant primary antibody (for a final concentration of 5µg/ml) and kept 1h at 4°C. Following a washing step with PBS+0.5% BSA, samples are incubated for 45' at 4°C with the correspondent secondary antibody (AlexaFluor 488 mouse, rabbit or goat diluted 1:1000). After a final washing step in PBS, samples are resuspended in 300µl PBS and analyzed at FACSCalibur (BD). Isotype-matched antibodies or secondary antibodies alone are used as negative control. Median fluorescence intensity of each sample is read using FLI channel and normalized for its negative control.

### Sample collection:

Inclusion criteria comprised only newly diagnosed case of cancer, none of the patients had previously received radio or chemotherapy treatment or underwent surgery before blood collection. All patients gave signed consent before included to the study. The study was conducted by Riga East university Hospital and was approved by a local ethical committee and it was conformed to Declaration of Helsinki. Blood have been collected in 10ml EDTA tubes, gently inverted and centrifuged at 1500g 10' RT in 30 minutes from the moment of the blood collection.

Blood center of North Estonia Hospital provided healthy certified donor plasma.

### Immunohistochemical examination of tissue

Tissue sections were immunostained to visualize cells that were positive for TM9SF4. Antigen retrieval was achieved by incubation the slides at Tris/EDTA buffer at pH=9.0 at scientific microwave for 30 min. Endogenous peroxidase activity was blocked with 3.0% H₂O₂ for 10 min. Aspecific primary antibody binding was blocked with normal horse serum prior to antibody incubation. The slides were incubated overnight at 4°C with rabbit polyclonal TM9SF4 antibody (dilution 1:400). The slides were incubated at room temperature for 1 hour at dilution 1:100. Antibodies binding was detected using the EnVision reagent (1 hour at room temperature). The immunoperoxidase reaction colour was developed by incubating the slides with diaminobenzidine for 7 min. A negative control that omitted the primary antibody was included for each experiment.

### Imaging and quantitation of cells.

For every specimen was given a score according to the intensity of the nucleic or cytoplasmic staining (no staining = 0, weak staining = 1, moderate staining = 2, strong staining = 3) and the extent of stained cells (0% = score 0; 1-10% = 1; 11-50% = 2; 51> = score 3. Negative means 0% area staining. Focally positive means 1-80% area staining, diffusely positive means 81-100% area staining. For Breast, Lung and Melanoma have been counted the number of cancers positive cells/mm2.

### Data analyses

The results for morphological data were expressed as the means ± SD. Morphological and immunohistochemical data were analysed by two-way ANOVA followed by Bonferroni post test for comparison between the groups. The correlation with clinical and histopathological data was assessed by Spearman test. In all tests, p value of < 0.05 was considered statistically significant. SPSS 21. version software was used for the statistical analysis.

### Standard protocol for protein markers detection by sandwich ELISA assay:

*ELISA assay for purified exosomes by ultracentrifugated conditioned media:* 40, 20, 10 and 5 µg/100µl PBS of isolated exosomes and 100µl of PBS as negative control (0 µg) are loaded onto a 96 well plate pre-coated with TM9SF4 (2 µg/ml) antibody (transparent plate). Briefly, 96 well plates are pre-coated with the relevant capture antibody, washed thrice with PBS+0.05%TWEEN (washing buffer), added with the isolated exosomes, and incubated overnight at 37°C. After three washes with washing buffer, the plates are incubated with CD9 detection antibody, incubated for 2 hrs at 37°C, washed thrice with washing buffer, incubated for one hour at 37°C with the corresponded secondary antibody and washed thrice with washing buffer. 100 µl TMB (tetramethylbenzidine) are added to each well and after 5 minutes the reaction is stopped by addition of 100 µl of stop solution (IN sulfuric acid).

The O/D absorbance is read with a M1000 Tecan at 450 nm.

### ELISA assay for biological fluids (plasma and serum):

Plasma and serum samples are stored at -80°C, thawed at room temperature and pre-cleared after the addition of 1:500 protease inhibitors cocktail centrifuging at 1200g 20' 4°C, transferring the supernatant in another vial and centrifuging again at 10000g 30' at 4°C. The supernatant obtained is called pre-cleared and is used for the following analysis.

Briefly 100 µl of pre-cleared plasma or serum are incubated overnight at 4°C in 96 well plates pre-coated with TM9SF4 antibody (2 µg/ml). After three washes with washing buffer, the plates are incubated with CD9 detection antibody, incubated for 2 hrs at 4°C, washed thrice with washing buffer, incubated for one hour at 4°C with the corresponded secondary antibody and washed thrice with washing buffer. 100 µl TMB (tetramethylbenzidine) are added to each well and after 5 minutes the reaction is stopped by addition of 100 µl of stop solution (IN sulfuric acid).

The O/D absorbance is read with a M1000 Tecan at 450 nm.

### Preparation of TM9SF4 coated beads

Beads coated with a TM9SF4 antibody can be obtained by using method known to the skilled man in that art or modifications thereof.

### RNA and miRNA extraction from immunocaptured exosomes.

### Exosome isolation by immunocapture through TM9SF4 pre-coated beads: culture media or biological fluids (plasma and serum)

10 mL supernatant from cell culture are added with Protease inhibitors diluted at 1:1000 and concentrated 10X using Centrifugal Filter Units (Millipore). 1ml 10X medium is then incubated overnight at 4°C with immunobeads pre-coated with TM9SF4 antibody.

Immunocaptured EVs are washed thrice with PBS + Tween 0.01%, and treated with 0.7ml QIAZOL.

100 µl of pre-cleared plasma or serum are diluted with 900 µl of PBS 1X and incubated overnight at 4°C in a rotator with 10 µl of TM9SF4 pre-coated beads. Beads are washed thrice with PBS + Tween 0.01% and treated with 0.7 ml QIAZOL.

Total RNA is extracted using Total RNA extraction kit (Hansabiomed) and eluted RNA is quantified at Nanodrop.

### miRNA and snoRNA amplification and RT-qPCR analysis

miRNA were retro-transcribed using a miScript II RT Kit (Qiagen) and 0.3 ng cDNA were amplified by qRT-PCR in CFX96™ real-time PCR detection system (BIORAD) with miScript SYBR Green PCR kit (Qiagen), using miScript primer assays (Qiagen) targeting miR-21 (Cat. Num: MS00009079), RNU6, (Cat. Num: MS00033740) and the reference miRNAs, miR-451 (Cat. Num.: MS00004242), miR-574 (Cat. Num.: MS00032025) and miR-223 (Cat. Num.: MS00003871).

### References cited

1. Carroll SL, Ratner N. How does the Schwann cell lineage form tumors in NF1? Glia. 2008;56(14):1590-605. Epub 2008/09/23. doi: 10.1002/glia.20776. PubMed PMID: 18803326; PubMed Central PMCID: PMC2652636.
2. Evans DG, Baser ME, McGaughran J, Sharif S, Howard E, Moran A. Malignant peripheral nerve sheath tumours in neurofibromatosis 1. J Med Genet. 2002;39(5):311-4. Epub 2002/05/16. PubMed PMID: 12011145; PubMed Central PMCID: PMC1735122.
3. Korf BR. Malignancy in neurofibromatosis type 1. Oncologist 2000;5(6):477-85.
4. Lewis JJ, Brennan MF. Soft tissue sarcomas. Curr Probl Surg. 1996;33(10):817-72. Epub 1996/10/01. PubMed PMID: 8885853.
5. Woodruff JMK, H.P.; Louis,D.N.; Scheithauer,B.W. Malignant peripheral nerve sheath tumour (MPNST). In: Kleihues PC, W.K., editor. Pathology and Genetics of Tumours of the Nervous System. First ed. Lyon: IARC Press; 2000. p. 172-4.
6. Ducatman BS, Scheithauer BW, Piepgras DG, Reiman HM, Ilstrup DM. Malignant peripheral nerve sheath tumors. A clinicopathologic study of 120 cases. Cancer. 1986;57(10):2006-21. Epub 1986/05/15. PubMed PMID: 3082508.
7. Ferner RE, Gutmann DH. International consensus statement on malignant peripheral nerve sheath tumors in neurofibromatosis. Cancer Res. 2002;62(5): 1573-7. Epub 2002/03/16. PubMed PMID: 11894862.
8. McQueen M, MacCollin M, Gusella J, Plotkin SR. Patient and physician attitudes regarding clinical trials in neurofibromatosis 1. J Neurosci Nurs. 2008;40(6):341-5. Epub 2009/01/28. PubMed PMID: 19170300.
9. Mathivanan S, Ji H, Simpson RJ (2010) Exosomes: extracellular organelles important in intercellular communication. J Proteomics 73: 1907-1920.
10. Keller S, Sanderson MP, Stoeck A, Altevogt P. Exosomes: from biogenesis and secretion to biological function. Immunol Lett. 2006 Nov 15;107(2):102-8. Epub 2006 Oct 17.
11. Simons M, Raposo G. Exosomes--vesicular carriers for intercellular communication. CurrOpinCell Biol. 2009;21(4):575-81.
12. Simpson RJ, Jensen SS, Lim JW. Proteomic profiling of exosomes: current perspectives. Proteomics. 2008 Oct;8(19):4083-99. doi: 10.1002/pmic.200800109.
13. Mathivanan S, Lim JW, Tauro BJ, Ji H, Moritz RL, Simpson RJ. Proteomics analysis of A33 immunoaffinity-purified exosomes released from the human colon tumor cell line LIM1215 reveals a tissue-specific protein signature. MolCell Proteomics. 2010;9(2):197-208.
14. Valadi H, Ekstrom K, Bossios A, Sjostrand M, Lee JJ, Lotvall JO. Exosome-mediated transfer of mRNAs and microRNAs is a novel mechanism of genetic exchange between cells. NatCell Biol. 2007;9(6):654-9.
15. Caby MP, Lankar D, Vincendeau-Scherrer C, Raposo G, Bonnerot C. Exosomal-like vesicles are present in human blood plasma. Int Immunol. 2005 Jul;17(7):879-87.
16. Mitchell PJ, Welton J, Staffurth J, Court J, Mason MD, Tabi Z, Clayton A. Can urinary exosomes act as treatment response markers in prostate cancer? 12;7:4. doi: 10.1186/1479-5876-7-4.
17. Skog J, Wurdinger T, van Rijn S, Meijer DH, Gainche L, Sena-Esteves M, et al. Glioblastoma microvesicles transport RNA and proteins that promote tumour growth and provide diagnostic biomarkers. NatCell Biol. 2008;10(12):1470-6.
18. Logozzi M, De Milito A, Lugini L, Borghi M, Calabro L, Spada M, Perdicchio M, Marino ML, Federici C, Iessi E, Brambilla D, Venturi G, Lozupone F, Santinami M, Huber V, Maio M, Rivoltini L, Fais S. High levels of_exosomes_expressing CD63 and caveolin-1 in plasma of melanoma patients. PLoS One. 2009;4(4):e5219.
19. Duijvesz D, Luider T, Bangma CH, Jenster G. Exosomes_as biomarker treasure_chests_for prostate cancer. Eur Urol. 2011 May;
20. Vlassov AV, Magdaleno S, Setterquist R, Conrad R. Exosomes: current knowledge of their composition, biological functions, and diagnostic and therapeutic potentials. Biochim Biophys Acta. 2012 Jul;1820(7):940-8.
21. Corrado C, Raimondo S, Chiesi A, Ciccia F, De Leo G, Alessandro R. Exosomes_as intercellular signaling organelles involved in health and disease: basic science and clinical applications. Int J Mol Sci. 2013 Mar 6;14(3):5338-66.
22. Chluba-de Tapia J, de Tapia M, Jäggin V, Eberle AN. Cloning of a human multispanning membrane protein cDNA: evidence for a new protein family. Gene. 1997 Sep 15;197(1-2):195-204.
23. Lozupone F, Perdicchio M, Brambilla D, Borghi M, Meschini S, Barca S, Marino ML, Logozzi M, Federici C, Iessi E, de Milito A, Fais S. The human homologue of Dictyostelium discoideum phg1A is expressed by human metastatic melanoma cells. EMBO Rep. 2009 Dec;10(12):1348-54. doi: 10.1038/embor.2009.236. Epub 2009 Nov 6.
24. Mackinnon RN, Selan C, Wall M, Baker E, Nandurkar H, Campbell LJ. The paradox of 20q11.21 amplification in a subset of cases of myeloid malignancy with chromosome 20 deletion. Genes Chromosomes Cancer. 2010 Nov;49(11):998-1013. doi: 10.1002/gcc.20806.
25. Fais S. Proton pump inhibitor-induced tumour cell death by inhibition of a detoxification mechanism. J Intern Med. 2010 May;267(5):515-25. doi: 10.1111/j.1365-2796.2010.02225.x.
26. Perrin J, Mortier M, Jacomin AC, Viargues P, Thevenon D, Fauvarque MO. The nonaspanins TM9SF2 and TM9SF4 regulate the plasma membrane localization and signalling activity of the peptidoglycan recognition protein PGRP-LC in Drosophila. J Innate Immun. 2015;7(1):37-46. doi: 10.1159/000365112. Epub 2014 Aug 13.
27. Caruso RA, Fedele F, Finocchiaro G, Arena G, Venuti A. Neutrophil-tumor cell phagocytosis (cannibalism) in human tumors: an update and literature review. Exp Oncol. 2012 34:306-11.
28. Caruso RA, Muda AO, Bersiga A, Rigoli L, Inferrera C. Morphological evidence of neutrophil-tumor cell phagocytosis (cannibalism) in human gastric adenocarcinomas. Ultrastruct Pathol. 2002 26:315-21.
29. McBurney McBurney MI, Van Soest PJ, Jeraci JL. Colonic carcinogenesis: the microbial feast or famine mechanism. Nutr Cancer. 1987;10(1-2):23-8.
30. Bansal C, Tiwari V, Singh U, Srivastava A, Misra J. Cell Cannibalism: A cytological study in effusion samples. J Cytol. 2011 28:57-60.
31. Lozupone F, Borghi M, Marzoli F, Azzarito T, Matarrese P, Iessi E, Venturi G, Meschini S, Canitano A, Bona R, Cara A, Fais S. TM9SF4 is a novel V-ATPase-interacting protein that modulates tumor pH alterations associated with drug resistance and invasiveness of colon cancer cells. Oncogene. 2015 Feb 9. doi: 10.1038/onc.2014.437. [Epub ahead of print]
32. Ambros V, Lee RC, Lavanway A, Williams PT, Jewell D. MicroRNAs and other tiny endogenous RNAs in C. elegans. Curr Biol. 2003;13:807-818.
33. Jazbutyte V, Thum T. MicroRNA-21: From cancer to cardiovascular disease. Curr Drug Targets. 2010;11:926-935.
34. Esquela-Kerscher A, Slack FJ. Oncomirs-microRNAs with a role in cancer. Nat Rev Cancer. 2006;6:259-269. [PubMed]
35. Zhang W, Dahlberg JE, Tam W. MicroRNAs in tumor-igenesis: A primer. Am J Pathol. 2007;171:728-738. [PMC free article] [PubMed]
36. Chan JA, Krichevsky AM, Kosik KS. MicroRNA-21 is an antiapoptotic factor in human glioblastoma cells. Cancer Res. 2005;65:6029-6033.
37. Lim QE, Zhou L, Ho YK, Too HP. snoU6 and 5S RNAs are not reliable miRNA reference genes in neuronal differentiation. Neuroscience 2011;199:32-43.

## Claims

1. A method for determining in vitro the presence of a tumour in a subject, such method comprising
a) providing a biological sample obtained from that subject,
b) isolating extracellular vesicles from said sample, wherein this step of isolating extracellular vesicles comprises isolating TM9SF4-positive extracellular vesicles ,
c) determining, from the extracellular vesicles isolated in step b), the level or presence of a suitable biomarker, and
d) comparing the level or presence of the biomarker determined in step c) with one or more reference values,
**characterized in that** the TM9SF4-positive extracellular vesicles are isolated through binding to an anti-TM9SF4 antibody.

2. The method according to claim 1, wherein at least a portion of the extracellular vesicles are exosomes.

3. The method according to claims 1-2, wherein the tumour is selected from the list consisting of colon cancer, gastric cancer, breast cancer, lung cancer, melanoma, pancreatic cancer, ovary cancer, prostate cancer, central nervous system tumour, glioblastoma or MPNST.

4. The method according to claims 1-3, wherein the biomarker of step c) is selected from the list consisting of CD9 protein, miR-21 or RNU6.

5. A method for determining in vitro the tumour transformation status in a subject, such method comprising:
a) providing a biological sample obtained from that subject,
b) isolating extracellular vesicles from said sample, wherein this step of isolating extracellular vesicles comprises isolating TM9SF4-positive extracellular vesicles,
c) determining, from the extracellular vesicles isolated in step b), the level or presence of a suitable biomarker, and
d) comparing the level or presence of the biomarker determined in step c) with one or more reference values,
**characterized in that** the TM9SF4-positive extracellular vesicles are isolated through binding to an anti-TM9SF4 antibody.

6. The method according to claim 5, wherein the biological sample of step a) is obtained from a subject affected by a benign tumour.

7. The method according to claims 5-6, wherein at least a portion of the extracellular vesicles are exosomes.

8. The method according to claim 5-6, wherein the tumour transformation status is the transformation to an MPNST or to a colorectal cancer.

9. The method according to claims 5-8, wherein the suitable biomarker is selected from the list consisting of CD9 protein, miR-21 or RNU6.

10. Use of TM9SF4-positive extracellular vesicles in a test to determine the presence of a tumour or the tumour transformation status in a subject, **characterized in that** the presence of the tumour or the tumour transformation status is determined by a method according to any of claims 1-9.

11. The use according to claim 10, wherein the extracellular vesicles are exosomes.

12. The use according to claims 10-11, wherein the tumour is selected from the list consisting of colon cancer, gastric cancer, breast cancer, lung cancer, melanoma, pancreatic cancer, ovary cancer, prostate cancer ,central nervous system tumour, glioblastoma or MPNST.

13. The use according to claims 10-11, wherein the tumour transformation status is the transformation to MPNST or to colorectal cancer.

14. Use of a kit to determine in an in vitro method according to any of claims 1-8 or 9 the presence of a tumour or a tumour transformation status in a subject, such kit comprising an anti-TM9SF4 antibody and a reagent selected from the list consisting of an anti CD9-antibody, a miR-21 primer or a RNU6 primer.

15. The use according to claim 14, wherein the tumour is selected form the list consisting of colon cancer, gastric cancer, breast cancer, lung cancer, melanoma, pancreatic cancer, ovary cancer, prostate cancer, central nervous system tumour, glioblastoma or MPNST.

16. The use according to claim 14, wherein the tumour transformation status is the transformation to an MPNST or to a colorectal cancer.

17. The use according to claims 14-16, further comprising instructions for suitable operational parameters in the form of a label or separate insert.

## Patentansprüche

1. Verfahren zur Bestimmung des Vorliegens eines Tumors in einem Lebewesen in vitro, wobei das Verfahren
a) Bereitstellen einer biologischen Probe, die von dem Lebewesen erhalten worden ist,
b) Isolieren von extrazellulären Vesikeln von der Probe, wobei dieser Schritt des Isolierens von extrazellulären Vesikeln das Isolieren von TM9SF4-positiven extrazellulären Vesikeln umfasst,
c) Bestimmen, aus den in dem Schritt b) isolierten extrazellulären Vesikeln, der Konzentration oder des Vorliegens eines geeigneten Biomarkers, und
d) Vergleichen der Konzentration oder des Vorliegens des im Schritt c) bestimmten Biomarkers mit einem oder mehreren Referenzwert(en), umfasst,
**dadurch gekennzeichnet, dass** die TM9SF4-positiven extrazellulären Vesikel durch Binden an einen anti-TM9SF4-Antikörper isoliert werden.

2. Verfahren nach Anspruch 1, bei dem mindestens ein Teil der extrazellulären Vesikel Exosomen sind.

3. Verfahren nach den Ansprüchen 1 bis 2, bei dem der Tumor aus der Liste, bestehend aus Kolonkrebs, Magenkrebs, Brustkrebs, Lungenkrebs, Melanom, Pankreaskrebs, Eierstockkrebs, Prostatakrebs, einem Tumor des zentralen Nervensystems, Glioblastom oder MPNST, ausgewählt ist.

4. Verfahren nach den Ansprüchen 1 bis 3, bei dem der Biomarker von Schritt c) aus der Liste, bestehend aus CD9-Protein, miR-21 oder RNU6, ausgewählt ist.

5. Verfahren zur Bestimmung des Tumortransformationsstatus in einem Lebewesen in vitro, wobei das Verfahren
a) Bereitstellen einer biologischen Probe, die von dem Lebewesen erhalten worden ist,
b) Isolieren von extrazellulären Vesikeln von der Probe, wobei dieser Schritt des Isolierens von extrazellulären Vesikeln das Isolieren von TM9SF4-positiven extrazellulären Vesikeln umfasst,
c) Bestimmen, aus den in dem Schritt b) isolierten extrazellulären Vesikeln, der Konzentration oder des Vorliegens eines geeigneten Biomarkers, und
d) Vergleichen der Konzentration oder des Vorliegens des im Schritt c) bestimmten Biomarkers mit einem oder mehreren Referenzwert(en), umfasst,
**dadurch gekennzeichnet, dass** die TM9SF4-positiven extrazellulären Vesikel durch Binden an einen anti-TM9SF4-Antikörper isoliert werden.

6. Verfahren nach Anspruch 5, bei dem die biologische Probe von Schritt a) von einem Lebewesen erhalten worden ist, das einen gutartigen Tumor aufweist.

7. Verfahren nach den Ansprüchen 5 bis 6, bei dem mindestens ein Teil der extrazellulären Vesikel Exosomen sind.

8. Verfahren nach den Ansprüchen 5 bis 6, bei dem der Tumortransformationsstatus die Transformation zu einem MPNST oder zu einem Kolorektalkrebs ist.

9. Verfahren nach den Ansprüchen 5 bis 8, bei dem der geeignete Biomarker aus der Liste, bestehend aus CD9-Protein, miR-21 oder RNU6, ausgewählt ist.

10. Verwendung von TM9SF4-positiven extrazellulären Vesikeln in einem Test zum Bestimmen des Vorliegens eines Tumors oder des Tumortransformationsstatus in einem Lebewesen, **dadurch gekennzeichnet, dass** das Vorliegen des Tumors oder der Tumortransformationsstatus durch ein Verfahren nach einem der Ansprüche 1 bis 9 bestimmt wird.

11. Verwendung nach Anspruch 10, bei der die extrazellulären Vesikel Exosomen sind.

12. Verwendung nach den Ansprüchen 10 bis 11, bei welcher der Tumor aus der Liste, bestehend aus Kolonkrebs, Magenkrebs, Brustkrebs, Lungenkrebs, Melanom, Pankreaskrebs, Eierstockkrebs, Prostatakrebs, einem Tumor des zentralen Nervensystems, Glioblastom oder MPNST, ausgewählt ist.

13. Verwendung nach den Ansprüchen 10 bis 11, bei welcher der Tumortransformationsstatus die Transformation zu einem MPNST oder zu einem Kolorektalkrebs ist.

14. Verwendung eines Kits zur Bestimmung des Vorliegens eines Tumors oder eines Tumortransformationsstatus in einem Lebewesen in einem vitro-Verfahren nach einem der Ansprüche 1 bis 8 oder 9, wobei das Kit einen anti-TM9SF4-Antikörper und ein Reagenz, ausgewählt aus der Liste, bestehend aus einem anti-CD9-Antikörper, einem miR-21-Primer oder einem RNU6-Primer, umfasst.

15. Verwendung nach Anspruch 14, bei welcher der Tumor aus der Liste, bestehend aus Kolonkrebs, Magenkrebs, Brustkrebs, Lungenkrebs, Melanom, Pankreaskrebs, Eierstockkrebs, Prostatakrebs, einem Tumor des zentralen Nervensystems, Glioblastom oder MPNST, ausgewählt ist.

16. Verwendung nach Anspruch 14, bei welcher der Tumortransformationsstatus die Transformation zu einem MPNST oder zu einem Kolorektalkrebs ist.

17. Verwendung nach den Ansprüchen 14 bis 16, die ferner Anweisungen für geeignete Ausführungsparameter in der Form eines Etiketts oder eines separaten Beipackzettels umfasst.

## Revendications

1. Procédé pour déterminer *in vitro* la présence d'une tumeur chez un sujet, un tel procédé comprenant
a) la fourniture d'un échantillon biologique obtenu à partir de ce sujet,
b) l'isolation des vésicules extracellulaires à partir dudit échantillon, dans lequel cette étape d'isolation des vésicules extracellulaires comprend l'isolation des vésicules extracellulaires TM9SF4-positives,
c) la détermination, à partir des vésicules extracellulaires isolées dans l'étape b), du taux ou de la présence d'un biomarqueur approprié, et
d) la comparaison du taux ou de la présence du biomarqueur déterminé dans l'étape c) à une ou plusieurs valeurs de référence,
**caractérisé en ce que** les vésicules extracellulaires TM9SF4-positives sont isolées par l'intermédiaire d'une liaison à un anticorps anti-TM9SF4.

2. Procédé selon la revendication 1, dans lequel au moins une partie des vésicules extracellulaires sont des exosomes.

3. Procédé selon les revendications 1 ou 2, dans lequel la tumeur est choisie dans la liste consistant en un cancer du côlon, un cancer gastrique, un cancer du sein, un cancer du poumon, un mélanome, un cancer pancréatique, un cancer de l'ovaire, un cancer de la prostate, une tumeur du système nerveux central, un glioblastome ou un MPNST.

4. Procédé selon les revendications 1 à 3, dans lequel le biomarqueur de l'étape c) est choisi dans la liste consistant en la protéine CD9, miR-21 ou RNU6.

5. Procédé pour la détermination *in vitro* de l'état de transformation tumorale chez un sujet, un tel procédé comprenant :
a) la fourniture d'un échantillon biologique obtenu à partir de ce sujet,
b) l'isolation des vésicules extracellulaires à partir dudit échantillon, dans lequel cette étape d'isolation des vésicules extracellulaires comprend l'isolation des vésicules extracellulaires TM9SF4-positives,
c) la détermination, à partir des vésicules extracellulaires isolées dans l'étape b), du taux ou de la présence d'un biomarqueur approprié, et
d) la comparaison du taux ou de la présence du biomarqueur déterminé dans l'étape c) à une ou plusieurs valeurs de référence,
**caractérisé en ce que** les vésicules extracellulaires TM9SF4-positives sont isolées par l'intermédiaire d'une liaison à un anticorps anti-TM9SF4.

6. Procédé selon la revendication 5, dans lequel l'échantillon biologique de l'étape a) est obtenu à partir d'un sujet touché par une tumeur bénigne.

7. Procédé selon les revendications 5 ou 6, dans lequel au moins une partie des vésicules extracellulaires sont des exosomes.

8. Procédé selon les revendications 5 ou 6, dans lequel l'état de transformation tumorale est la transformation en un MPNST ou en un cancer colorectal.

9. Procédé selon les revendications 5 à 8, dans lequel le biomarqueur approprié est choisi dans la liste consistant en la protéine CD9, miR-21 ou RNU6.

10. Utilisation de vésicules extracellulaires TM9SF4-positives dans un test pour déterminer la présence d'une tumeur ou l'état de transformation tumorale chez un sujet, **caractérisée en ce que** la présence de la tumeur ou l'état de transformation tumorale est déterminé par un procédé selon l'une quelconque des revendications 1 à 9.

11. Utilisation selon la revendication 10, dans laquelle les vésicules extracellulaires sont des exosomes.

12. Utilisation selon les revendications 10 ou 11, dans laquelle la tumeur est choisie dans la liste consistant en un cancer du côlon, un cancer gastrique, un cancer du sein, un cancer du poumon, un mélanome, un cancer pancréatique, un cancer de l'ovaire, un cancer de la prostate, une tumeur du système nerveux central, un glioblastome ou un MPNST.

13. Utilisation selon les revendications 10 ou 11, dans laquelle l'état de transformation tumorale est la transformation en un MPNST ou en un cancer colorectal.

14. Utilisation d'un kit pour déterminer dans un procédé *in vitro* selon l'une quelconque des revendications 1 à 8 ou 9 la présence d'une tumeur ou d'un état de transformation tumorale chez un sujet, un tel kit comprenant un anticorps anti-TM9SF4 et un réactif choisi dans la liste consistant en un anticorps anti-CD9, une amorce miR-21 ou une amorce RNU6.

15. Utilisation selon la revendication 14, dans laquelle la tumeur est choisie dans la liste consistant en un cancer du côlon, un cancer gastrique, un cancer du sein, un cancer du poumon, un mélanome, un cancer pancréatique, un cancer de l'ovaire, un cancer de la prostate, une tumeur du système nerveux central, un glioblastome ou un MPNST.

16. Utilisation selon la revendication 14, dans laquelle l'état de transformation tumorale est la transformation en un MPNST ou en un cancer colorectal.

17. Utilisation selon les revendications 14 à 16, comprenant en outre des instructions pour les paramètres opérationnels appropriés sous la forme d'une étiquette ou d'une notice séparée.


### SEQUENCE LISTING

<110> HANSABIOMED OU
<120> EXOSOMAL BIOMARKERS
<130> PC1375EC
<160> 4
<170> PatentIn version 3.5
<210> 1
   <211> 642
   <212> PRT
   <213> Homo sapiens
<400> 1
   Met Ala Thr Ala Met Asp Trp Leu Pro Trp Ser Leu Leu Leu Phe Ser
   1               5                   10                  15
   Leu Met Cys Glu Thr Ser Ala Phe Tyr Val Pro Gly Val Ala Pro Ile
               20                  25                  30
   Asn Phe His Gln Asn Asp Pro Val Glu Ile Lys Ala Val Lys Leu Thr
           35                  40                  45
   Ser Ser Arg Thr Gln Leu Pro Tyr Glu Tyr Tyr Ser Leu Pro Phe Cys
       50                  55                  60
   Gln Pro Ser Lys Ile Thr Tyr Lys Ala Glu Asn Leu Gly Glu Val Leu
   65                  70                  75                  80
   Arg Gly Asp Arg Ile Val Asn Thr Pro Phe Gln Val Leu Met Asn Ser
                   85                  90                  95
   Glu Lys Lys Cys Glu Val Leu Cys Ser Gln Ser Asn Lys Pro Val Thr
               100                 105                 110
   Leu Thr Val Glu Gln Ser Arg Leu Val Ala Glu Arg Ile Thr Glu Asp
           115                 120                 125
   Tyr Tyr Val His Leu Ile Ala Asp Asn Leu Pro Val Ala Thr Arg Leu
       130                 135                 140
   Glu Leu Tyr Ser Asn Arg Asp Ser Asp Asp Lys Lys Lys Glu Lys Asp
   145                 150                 155                 160
   Val Gln Phe Glu His Gly Tyr Arg Leu Gly Phe Thr Asp Val Asn Lys
                   165                 170                 175
   Ile Tyr Leu His Asn His Leu Ser Phe Ile Leu Tyr Tyr His Arg Glu
               180                 185                 190
   Asp Met Glu Glu Asp Gln Glu His Thr Tyr Arg Val Val Arg Phe Glu
           195                 200                 205
   Val Ile Pro Gln Ser Ile Arg Leu Glu Asp Leu Lys Ala Asp Glu Lys
       210                 215                 220
   Ser Ser Cys Thr Leu Pro Glu Gly Thr Asn Ser Ser Pro Gln Glu Ile
   225                 230                 235                 240
   Asp Pro Thr Lys Glu Asn Gln Leu Tyr Phe Thr Tyr Ser Val His Trp
                   245                 250                 255
   Glu Glu Ser Asp Ile Lys Trp Ala Ser Arg Trp Asp Thr Tyr Leu Thr
               260                 265                 270
   Met Ser Asp Val Gln Ile His Trp Phe Ser Ile Ile Asn Ser Val Val
           275                 280                 285
   Val Val Phe Phe Leu Ser Gly Ile Leu Ser Met Ile Ile Ile Arg Thr
       290                 295                 300
   Leu Arg Lys Asp Ile Ala Asn Tyr Asn Lys Glu Asp Asp Ile Glu Asp
   305                 310                 315                 320
   Thr Met Glu Glu Ser Gly Trp Lys Leu Val His Gly Asp Val Phe Arg
                   325                 330                 335
   Pro Pro Gln Tyr Pro Met Ile Leu Ser Ser Leu Leu Gly Ser Gly Ile
               340                 345                 350
   Gln Leu Phe Cys Met Ile Leu Ile Val Ile Phe Val Ala Met Leu Gly
           355                 360                 365
   Met Leu Ser Pro Ser Ser Arg Gly Ala Leu Met Thr Thr Ala Cys Phe
       370                 375                 380
   Leu Phe Met Phe Met Gly Val Phe Gly Gly Phe Ser Ala Gly Arg Leu
   385                 390                 395                 400
   Tyr Arg Thr Leu Lys Gly His Arg Trp Lys Lys Gly Ala Phe Cys Thr
                   405                 410                 415
   Ala Thr Leu Tyr Pro Gly Val Val Phe Gly Ile Cys Phe Val Leu Asn
               420                 425                 430
   Cys Phe Ile Trp Gly Lys His Ser Ser Gly Ala Val Pro Phe Pro Thr
           435                 440                 445
   Met Val Ala Leu Leu Cys Met Trp Phe Gly Ile Ser Leu Pro Leu Val
       450                 455                 460
   Tyr Leu Gly Tyr Tyr Phe Gly Phe Arg Lys Gln Pro Tyr Asp Asn Pro
   465                 470                 475                 480
   Val Arg Thr Asn Gln Ile Pro Arg Gln Ile Pro Glu Gln Arg Trp Tyr
                   485                 490                 495
   Met Asn Arg Phe Val Gly Ile Leu Met Ala Gly Ile Leu Pro Phe Gly
               500                 505                 510
   Ala Met Phe Ile Glu Leu Phe Phe Ile Phe Ser Ala Ile Trp Glu Asn
           515                 520                 525
   Gln Phe Tyr Tyr Leu Phe Gly Phe Leu Phe Leu Val Phe Ile Ile Leu
       530                 535                 540
   Val Val Ser Cys Ser Gln Ile Ser Ile Val Met Val Tyr Phe Gln Leu
   545                 550                 555                 560
   Cys Ala Glu Asp Tyr Arg Trp Trp Trp Arg Asn Phe Leu Val Ser Gly
                   565                 570                 575
   Gly Ser Ala Phe Tyr Val Leu Val Tyr Ala Ile Phe Tyr Phe Val Asn
               580                 585                 590
   Lys Leu Asp Ile Val Glu Phe Ile Pro Ser Leu Leu Tyr Phe Gly Tyr
           595                 600                 605
   Thr Ala Leu Met Val Leu Ser Phe Trp Leu Leu Thr Gly Thr Ile Gly
       610                 615                 620
   Phe Tyr Ala Ala Tyr Met Phe Val Arg Lys Ile Tyr Ala Ala Val Lys
   625                 630                 635                 640
   Ile Asp
<210> 2
   <211> 228
   <212> PRT
   <213> Homo sapiens
<400> 2
   Met Pro Val Lys Gly Gly Thr Lys Cys Ile Lys Tyr Leu Leu Phe Gly
   1               5                   10                  15
   Phe Asn Phe Ile Phe Trp Leu Ala Gly Ile Ala Val Leu Ala Ile Gly
               20                  25                  30
   Leu Trp Leu Arg Phe Asp Ser Gln Thr Lys Ser Ile Phe Glu Gln Glu
           35                  40                  45
   Thr Asn Asn Asn Asn Ser Ser Phe Tyr Thr Gly Val Tyr Ile Leu Ile
       50                  55                  60
   Gly Ala Gly Ala Leu Met Met Leu Val Gly Phe Leu Gly Cys Cys Gly
   65                  70                  75                  80
   Ala Val Gln Glu Ser Gln Cys Met Leu Gly Leu Phe Phe Gly Phe Leu
                   85                  90                  95
   Leu Val Ile Phe Ala Ile Glu Ile Ala Ala Ala Ile Trp Gly Tyr Ser
               100                 105                 110
   His Lys Asp Glu Val Ile Lys Glu Val Gln Glu Phe Tyr Lys Asp Thr
           115                 120                 125
   Tyr Asn Lys Leu Lys Thr Lys Asp Glu Pro Gln Arg Glu Thr Leu Lys
       130                 135                 140
   Ala Ile His Tyr Ala Leu Asn Cys Cys Gly Leu Ala Gly Gly Val Glu
   145                 150                 155                 160
   Gln Phe Ile Ser Asp Ile Cys Pro Lys Lys Asp Val Leu Glu Thr Phe
                   165                 170                 175
   Thr Val Lys Ser Cys Pro Asp Ala Ile Lys Glu Val Phe Asp Asn Lys
               180                 185                 190
   Phe His Ile Ile Gly Ala Val Gly Ile Gly Ile Ala Val Val Met Ile
           195                 200                 205
   Phe Gly Met Ile Phe Ser Met Ile Leu Cys Cys Ala Ile Arg Arg Asn
       210                 215                 220
   Arg Glu Met Val
   225
<210> 3
   <211> 22
   <212> RNA
   <213> Homo sapiens
<400> 3
   uagcuuauca gacugauguu ga 22
<210> 4
   <211> 107
   <212> DNA
   <213> Homo sapiens
<400> 4
   gtgctcgctt cggcagcaca tatactaaaa ttggaacgat acagagaaga ttagcatggc 60
   ccctgcgcaa ggatgacacg caaattcgtg aagcgttcca tattttt 107